# EUROPEAN PATENT SPECIFICATION

(11) **EP 3 966 830 B1**
(45) Date of publication and mention of the grant of the patent: **04.11.2026**
(21) Application number: 20720468.6
(22) Date of filing: 28.04.2020
(51) Int. Cl.: G16H 30/40, G16H 50/20, G16H 50/70

(54) **IDENTIFICATION OF CANDIDATE SIGNS INDICATIVE OF AN NTRK ONCOGENIC FUSION**
IDENTIFIZIERUNG VON KANDITATENZEICHEN, DIE AUF EINE NTRK-ONKOGENE FUSION HINDEUTEN
IDENTIFICATION DE SIGNES CANDIDATS INDIQUANT UNE FUSION ONCOGÈNE NTRK

(30) Priority: 10.05.2019 EP 19173832
(43) Date of publication of application: 16.03.2022
(73) Proprietor: Bayer Consumer Care AG, 4052 Basel (CH)
(72) Inventor: SCHMITZ, Arndt, 12167 Berlin (DE); ELCI, Eren, Metin, 64625 Bensheim (DE); STAVROPOULOU, Faidra, 10439 Berlin (DE); KACHALA, Mikhail, 50968 Köln (DE); KARLSSON, Antti, 20780 Kaarina (FI); TUKIAINEN, Mikko, 20720 Turku (FI)
(74) Representative: BIP Patents
(86) International application number: PCT/EP2020/061665
(87) International publication number: WO 2020/229152

(56) References cited:
- WO-A1-2019/084697
- FRÉDÉRIQUE PENAULT-LLORCA ET AL: "Testing algorithm for identification of patients with TRK fusioncancer Best practice", J CLIN PATHOL, vol. 72, 9 May 2019 (2019-05-09), pages 460 - 467, XP055639828, DOI: 10.1136/jclinpath-2018-205679
- JESSICA L DAVIS ET AL: "Infantile NTRK -associated Mesenchymal Tumors", PEDIATRIC AND DEVELOPMENTAL PATHOLOGY, vol. 21, no. 1, 6 July 2017 (2017-07-06), US, pages 68 - 78, XP055639805, ISSN: 1093-5266, DOI: 10.1177/1093526617712639
- COUDRAY NICOLAS ET AL: "Classification and mutation prediction from non-small cell lung cancer histopathology images using deep learning", NATURE MEDICINE, NATURE PUB. CO, NEW YORK, vol. 24, no. 10, 17 September 2018 (2018-09-17), pages 1559 - 1567, XP036608997, ISSN: 1078-8956, [retrieved on 20180917], DOI: 10.1038/S41591-018-0177-5
- HARISH BABU ARUNACHALAM ET AL: "Viable and necrotic tumor assessment from whole slide images of osteosarcoma using machine-learning and deep-learning models", PLOS ONE, vol. 14, no. 4, 17 April 2019 (2019-04-17), pages e0210706, XP055639915, DOI: 10.1371/journal.pone.0210706

## Description

The present invention relates to the identification of one or more candidate signs indicative of an NTRK oncogenic fusion within patient data. Subject matter of the present invention are a computer-implemented method, a system, and a non-transitory computer-readable storage medium for determining a probability value from patient data associated with a subject patient, the probability value indicating the probability of the subject patient suffering from cancer caused by a mutation of a neurotrophic receptor tyrosine kinase (NTRK) gene.

The tropomyosin receptor kinase (Trk) receptor family comprises three transmembrane proteins referred to as Trk A, B and C (TrkA, TrkB and TrkC) receptors, and are encoded by the NTRK1, NTRK2 and NTRK3 genes, respectively.

The wildtype forms of these receptor tyrosine kinases are expressed in human neuronal tissue, and play an essential role in both the physiology of development and function of the nervous system through activation by neurotrophins.

NTRK genes, similar to other genes, are subject to alterations, including fusions. Preclinical studies have demonstrated that Trk fusion proteins promote oncogenesis by mediating constitutive cell proliferation and survival.

NTRK oncogenic fusions arise from intra-chromosomal or inter-chromosomal rearrangements that juxtapose the kinase domain-containing 3' region of NTRK with the 5' region of NTRK's gene partner.

NTRK oncogenic fusions are infrequent but recurrent events observed in various types of congenital and acquired cancers (see e.g. Table 2 of Ed S. Kheder, David S. Hong: Emerging Targeted Therapy for Tumors with NTRK Fusion Proteins, clincancerres.aacrjournals.org, 2018, DOI: 10.1158/1078-0432.CCR-18-1156).

These genetic abnormalities have recently emerged as targets for cancer therapy, because novel compounds have been developed that are selective inhibitors of the constitutively active rearranged proteins. Over the past few years, various inhibitors targeting the Trk family members have been developed and tested in clinical trials (see e.g. Table 3 of Ed S. Kheder, David S. Hong: Emerging Targeted Therapy for Tumors with NTRK Fusion Proteins, clincancerres.aacrjournals.org, 2018, DOI: 10.1158/1078-0432.CCR-18-1156). Specifically, larotrectinib and entrectinib have emerged as potent, safe, and promising Trk inhibitors.

A major challenge in the development of these inhibitors and their use as therapeutics is the low incidence in each single tumor histology.

Next-generation sequencing provides a precise method to detect NTRK gene fusions (M.L. Metzker: Sequencing technologies - the next generation, Nat Rev Genet. 2010, 11(1), pages 31-46). However, performing gene analyses for each patient is expensive and, due to the low incidence of NTRK oncogenic fusions, inefficient.

Immunohistochemistry provides a routine method to detect protein expression of NTRK genes (for example, J. F. Hechtman et al.: Pan-Trk Immunohistochemistry Is an Efficient and Reliable Screen for the Detection of NTRK Fusions, Am J Surg Pathol. 2017, 41 (11), pages 1547 - 1551; https://www.prnewswire.com/news-releases/roche-launches-first-ivd-pan-trk-immunohistochemistry-assay-300755647.html). However, performing immunohistochemistry requires skills and the correlation between protein expression and gene fusion status is not trivial. Interpretation of IHC results requires the skills of a trained and certified medical professional pathologist. Similar practical challenges also hold true for other molecular assays such as FISH (fluorescence in situ hybridization) or PCR (polymerase chain reaction). FREDERIQUE PENAULT-LLORCA ET AL: "Testing algorithm for identification of patients with TRK fusioncancer Best practice", J CLIN PATHOL, vol. 72, 9 May 2019 (2019-05-09), pages 460-467, discuss the biology of NTRK gene fusions, and present a testing algorithm to aid detection of these gene fusions in clinical practice and guide treatment decisions.

The subject matter of the present invention addresses the problems identified above.

In a first aspect, the present invention provides a computer-implemented method for identifying one or more candidate signs indicative of an NTRK oncogenic fusion within patient data associated with a subject patient, the method comprising:
- receiving patient data of a subject patient suffering from cancer, the patient data comprising at least one histopathological image of tumor tissue of the subject patient;
- inputting the patient data into a prediction model trained via machine learning with a set of training data, the training data comprising histopathological images, the prediction model being configured for identifying within the patient data one or more characteristics of an NTRK oncogenic fusion;
- receiving as an output from the prediction model a probability value, the probability value indicating the probability of the subject patient suffering from cancer caused by an NTRK oncogenic fusion.

In a second aspect, the present invention provides a system, comprising:
a processor; and
a memory storing an application program configured to perform, when executed by the processor, an operation for identifying one or more candidate signs indicative of an NTRK oncogenic fusion within patient data associated with a subject patient, the operation comprising:
   - receiving patient data of a subject patient suffering from cancer, the patient data comprising at least one histopathological image of tumor tissue of the subject patient;
   - inputting the patient data into a prediction model trained via machine learning with a set of training data, the training data comprising histopathological images, the prediction model being configured for identifying within the patient data one or more characteristics of an NTRK oncogenic fusion;
   - receiving as an output from the prediction model a probability value, the probability value indicating the probability of the subject patient suffering from cancer caused by an NTRK oncogenic fusion.

In a third aspect, the present invention provides a non-transitory computer-readable storage medium comprising processor-executable instructions with which to perform an operation for identifying one or more candidate signs indicative of an NTRK oncogenic fusion within patient data associated with a subject patient, the operation comprising:
- receiving patient data of a subject patient suffering from cancer, the patient data comprising at least one histopathological image of tumor tissue of the subject patient;
- inputting the patient data into a prediction model trained via machine learning with a set of training data, the training data comprising histopathological images, the prediction model being configured for identifying within the patient data one or more characteristics of an NTRK oncogenic fusion;
- receiving as an output from the prediction model a probability value, the probability value indicating the probability of the subject patient suffering from cancer caused by an NTRK oncogenic fusion.

The invention will be more particularly elucidated below without distinguishing between the subjects of the invention. On the contrary, the following elucidations are intended to apply analogously to all subjects of the invention, irrespective of in which context they occur.

The present invention serves to determine a probability value from patient data associated with a subject patient by means of a prediction model, the probability value indicating the probability of the subject patient suffering from cancer caused by a mutation of a neurotrophic receptor tyrosine kinase (NTRK) gene.

The term "mutation" refers herein to any gene alteration including DNA rearrangement, nucleotide(s) substitution, addition and/or deletion that results in expression of a tropomyosin receptor kinase (Trk) receptor possessing an altered amino acid sequence. In particular, the term refers to gene alteration that juxtapose the kinase domain-containing 3' region of NTRK (NTRK1, NTRK2, NTRK3) with the 5' region of a NTRK's gene partner.

NTRK gene family fusion partners and associated cancers can be found e.g. in Ed S. Kheder, David S. Hong: Emerging Targeted Therapy for Tumors with NTRK Fusion Proteins, clincancerres.aacrjournals.org, 2018, DOI: 10.1158/1078-0432.CCR-18-1156 (see in particular Table 1).

For the NTRK1 gene the following gene fusion partners in connection with colorectal cancers are described:
- TPM3 (tropomyosin 3) (Ardini E, et al. The TPM3-NTRK1 rearrangement is a recurring event in colorectal carcinoma and is associated with tumor sensitivity to TRKA kinase inhibition. Mol Oncol. 2014;8(8):1495-507),
- LMNA (lamin A/C) (Sartore-Bianchi A et al. Sensitivity to Entrectinib Associated With a Novel LMNA-NTRK1 Gene Fusion in Metastatic Colorectal Cancer. J Natl Cancer Inst. 2016;108(1)),
- TPR (translocated promoter region) (Lee SJ et al. NTRK1 rearrangement in colorectal cancer patients: evidence for actionable target using patient-derived tumor cell line. Oncotarget. 2015;6(36):39028-35),
- SCYL3, (SCY1 like pseudokinase 3) (Milione M., et al. Identification and characterization of a novel SCYL3-NTRK1 rearrangement in a colorectal cancer patient. Oncotarget. 2017;8(33):55353-60.

For the NTRK1 gene the following gene fusion partners in connection with lung cancer are described:
- CD74 (Vaishnavi A, et al. Oncogenic and drug-sensitive NTRK1 rearrangements in lung cancer. Nat Med. 2013;19(11):1469-72),
- **MPRIP** (myosin phosphatase rho interacting protein) (Vaishnavi A, et al. Oncogenic and drug-sensitive NTRK1 rearrangements in lung cancer. Nat Med. 2013;19(11):1469-72),
- SQSTM1 (sequestosome 1) (Farago AF, et al. Durable Clinical Response to Entrectinib in NTRK1-Rearranged Non-Small Cell Lung Cancer. J Thorac Oncol. 2015;10(12):1670-4.).

For the NTRK2 gene the following gene fusion partners in connection with lung cancer are described:
- TRIM24 (tripartite motif containing 24) (Stransky N, et al., The landscape of kinase fusions in cancer. Nat Commun. 2014;5:4846).

For the NTRK1 gene the following gene fusion partners in connection with glioblastoma multiforme cancer are described:
- ARHGEF2 (rho/rac guanine nucleotide exchange factor 2) ( Zheng Z, et al. Anchored multiplex PCR for targeted next-generation sequencing. Nat Med. 2014;20(12):1479-84.),
- BCAN (brevican) (Kim J, et al. NTRK1 fusion in glioblastoma multiforme. PLoS One. 2014;9(3):e91940.),
- NFASC (neurofascin) (Frattini V, et al. The integrated landscape of driver genomic alterations in glioblastoma. Nat Genet. 2013;45(10):1141-9.),
- TPM3 (tropomyosin 3) (Wu G, et al. The genomic landscape of diffuse intrinsic pontine glioma and pediatric non-brainstem high-grade glioma. Nat Genet. 2014;46(5):444-50.).

For the NTRK3 gene the following gene fusion partners in connection with glioblastoma multiforme cancer are described:
- ETV6 (ets variant 6) (Zheng Z, et al. Anchored multiplex PCR for targeted next-generation sequencing. Nat Med. 2014;20(12):1479-84. and Frattini V., et al. The integrated landscape of driver genomic alterations in glioblastoma. Nat Genet. 2013;45(10):1141-9).

For the NTRK2 gene the following gene fusion partners in connection with pilocytic astrocytoma cancer are described:
- NACC2 (NACC family member 2) (Jones DT, et al. Recurrent somatic alterations of FGFR1 and NTRK2 in pilocytic astrocytoma. Nat Genet. 2013;45(8):927-32.),
- QKI (KH domain containing RNA binding) (Jones DT, et al. Recurrent somatic alterations of FGFR1 and NTRK2 in pilocytic astrocytoma. Nat Genet. 2013;45(8):927-32).

For the NTRK1 gene the following gene fusion partners in connection with spitzoid melanoma cancer are described:
- TP53 (tumor protein P53) (Wiesner T, et al. Kinase fusions are frequent in Spitz tumours and spitzoid melanomas. Nat Commun. 2014;5:3116.),
- LMNA (lamin A/C) (Wiesner T, , et al. Kinase fusions are frequent in Spitz tumours and spitzoid melanomas. Nat Commun. 2014;5:3116).

For the NTRK1 gene the following gene fusion partners in connection with papillary thyroid cancer are described:
- TPM3 (tropomyosin 3) (Bongarzone I, et al. High frequency of activation of tyrosine kinase oncogenes in human papillary thyroid carcinoma. Oncogene. 1989;4(12):1457-62)),
- TFG (TRK-fused gene) (Greco A, et al. The DNA rearrangement that generates the TRK-T3 oncogene involves a novel gene on chromosome 3 whose product has a potential coiled-coil domain. Mol Cell Biol. 1995;15(11):6118-27.),
- TPR (translocated promoter region) (Greco A, et al., TRK-T1 is a novel oncogene formed by the fusion of TPR and TRK genes in human papillary thyroid carcinomas. Oncogene.1992;7(2):237-42.)).

For the NTRK3 gene the following gene fusion partners in connection with mammary analogue secretory carcinoma cancer are described:
- ETV6 (ets variant 6) (Ito S, et al., Case report of Mammary Analog Secretory Carcinoma of the parotid gland. Pathol Int. 2012;62(2):149-52., Del Castillo et al. Secretory Breast Carcinoma: A Histopathologic and Genomic Spectrum Characterized by a Joint Specific ETV6-NTRK3 Gene Fusion. Am J Surg Pathol. 2015;39(11):1458-67).

For the NTRK3 gene the following gene fusion partners in connection with secretory breast carcinoma cancer are described:
- ETV6 (ets variant 6) (Tognon C., et al. Expression of the ETV6-NTRK3 gene fusion as a primary event in human secretory breast carcinoma. Cancer Cell. 2002;2(5):367-76).

For the NTRK1 gene the following gene fusion partners in connection with infantile fibrosarcoma cancer are described:
- LMNA (lamin A/C) (Wong V., et al. Evaluation of a Congenital Infantile Fibrosarcoma by Comprehensive Genomic Profiling Reveals an LMNA-NTRK1 Gene Fusion Responsive to Crizotinib. J Natl Cancer Inst. 2016;108(1)).

For the NTRK2 gene the following gene fusion partners in connection with head and neck squamous cell carcinoma cancer are described:
- PAN3 (poly(A) specific ribonuclease subunit) (Stransky N, et al., landscape of kinase fusions in cancer. Nat Commun. 2014;5:4846.).

For the NTRK3 gene the following gene fusion partners in connection with mesoblastic nephroma cancer are described:
- ETV6 (ets variant 6) (Anderson J, et al., Expression of ETV6-NTRK in classical, cellular and mixed subtypes of congenital mesoblastic nephroma. Histopathology. 2006;48(6):748-53).

For the NTRK3 gene the following gene fusion partners in connection with gastrointestinal stromal tumor cancer are described:
- ETV6 (ets variant 6) (Brenca M, et al. Transcriptome sequencing identifies ETV6-NTRK3 as a gene fusion involved in GIST. J Pathol. 2016;238(4):543-9., Shi E, et al. FGFR1 and NTRK3 actionable alterations in "Wild-Type" gastrointestinal stromal tumors. J Transl Med. 2016;14(1):339).

The probability value, indicating the probability of the cancer being caused by a mutation of a neurotrophic receptor tyrosine kinase (NTRK) gene, is determined from patient data. The patient data comprise at least one histopathological image of tumor tissue of the subject patient.

The at least one histopathological image can be obtained from the subject patient by biopsy.

In a preferred embodiment of the present invention, the at least one histopathological image is a microscopic image of tumor tissue of the subject patient. The magnification factor is preferably in the range of 10 to 60, more preferably in the range of 20 to 40, whereas a magnification factor of e.g. "20" means that a distance of 0.05 mm in the tumor tissue corresponds to a distance of 1 mm in the image (0.05 mm x 20 = 1 mm).

In a preferred embodiment of the present invention, the at least one histopathological image is a whole-slide image.

In a preferred embodiment of the present invention, the at least one histopathological image is an image of a stained tumor tissue sample. One or more dyes can be used to create the stained images. Preferred dyes are hematoxylin and eosin.

In a preferred embodiment of the present invention, the at least one histopathological image is a whole-slide image from a histopathological tissue slide of a stained tumor tissue sample.

Methods for creating histopathological images, in particular stained whole-slide microscopy images, are extensively described in scientific literature and textbooks (see e.g. S. K. Suvarna et al.: Bancroft's Theory and Practice of Histological Techniques, 8th Ed., Elsevier 2019, ISBN 978-0-7020-6864-5; A. F. Frangi et al.: Medical Image Computing and Computer Assisted Intervention - MICCAI 2018, 21st International Conference Granada, Spain, 2018 Proceedings, Part II, ISBN 978-030-00933-5; L.C. Junqueira et al.: Histologie, Springer 2001, ISBN: 978-354-041858-0; N. Coudray et al.: Classification and mutation prediction from non-small cell lung cancer histopathology images using deep learning, Nature Medicine, Vol. 24, 2018, pages 1559-1567).

The at least one histopathological image is a digital image. A digital image is a numeric representation, normally binary, of a two-dimensional image. Depending on whether the image resolution is fixed, it may be of vector or raster type. In a preferred embodiment of the present invention, the at least one histopathological image is a raster image holding RGB color values in 3 image channels. The RGB color model is an additive color model in which red (R), green (G) and blue (B) are added together in various ways to reproduce a broad array of colors. Alternatively, the at least one histopathological image is in Color Space Pixel (YUV) format having brightness, luminance, and color chrominance values. Other formats are conceivable as well.

Preferably the number of recorded pixels (pixel resolution) of the at least one histopathological image is in the range of 1,000 x 1,000 pixels to 500,000 x 500,000 pixels, more preferably in the range of 10,000 x 10,000 pixels to 100,000 to 100,000 pixels. The image can be quadratic or rectangular or of any other shape.

In a preferred embodiment, the patient data comprises more than one histopathological image. The histopathological images can be obtained e.g. at different magnifications (see e.g. WO2018/156133).

In a preferred embodiment, the at least one histopathological image is segmented into tumor regions and healthy regions. Tumor regions show tumor tissue. Healthy regions show healthy tissue. The segmentation is preferably done automatically using a trained convolutional neural network (see e.g. N. Coudray: Classification and mutation prediction from non-small cell lung cancer histopathology images using deep learning, Nature Medicine Vol 24, 1560, 2018, pages 1559-1567).

Depending on the sizes of the histopathological images used for training and validation of the prediction model as well as for prediction purposes on the one hand side, and the available computer power on the other hand side, the images can be divided into smaller preferably non-overlapping tiles. Training and validation of the prediction model as well as predictions of probability values is then preferably performed using tiles instead of the whole image. Preferably only those tiles are used which show tumor regions.

Additional patient data which can be used for the prediction of the probability value are anatomic or physiology data of the patient, such as information about patient's height and weight, gender, age, vital parameters (such as blood pressure, breathing frequency and heart rate), tumor grades, ICD-9 classification, oxygenation of tumor, degree of metastasis of tumor, blood count value tumor indicator value like PA value, information about the tissue the histopathological image is created from (e.g. tissue type, organ), further symptoms, medical history etc. Also, the pathology report of the histopathological images can be used for the prediction of the probability value, using text mining approaches. Also, a next generation sequencing raw data set which does not cover the TRK genes' sequences can be used for the prediction of the probability value.

Patient data can be e.g. collected from one or more databases or inputted into the system according to the present invention manually by a user.

The patient data is inputted into a prediction model. The prediction model uses one or more machine learning algorithms. A machine learning algorithm is an algorithm that can learn based on a set of training data. Embodiments of machine learning algorithms can be designed to model high-level abstractions within a data set. For example, image recognition algorithms can be used to determine which of several categories to which a given input belong; regression algorithms can output a numerical value given an input.

The prediction model can be or comprise an artificial neural network. The present invention preferably uses an artificial neural network comprising at least three layers of processing elements: a first layer with input neurons (nodes), an Nth layer with at least one output neuron (node), and N-2 inner layers, where N is a natural number greater than 2.

In such a network, the output neuron(s) serve(s) to predict at least one probability value, the probability value indicating the probability of the subject patient suffering from cancer caused by an NTRK oncogenic fusion. The input neurons serve to receive patient data as input values. Usually there is one input neuron for each pixel of the at least one histopathological image. There can be additional input neurons for the additional patient data as listed above.

The processing elements of the layers are interconnected in a predetermined pattern with predetermined connection weights therebetween. The network has been previously trained to identify one or more candidate signs indicative of an NTRK oncogenic fusion within the patient data. The training can be performed with a set of training data comprising patient data for which candidate signs indicative of an NTRK oncogenic fusion have been verified or excluded by means of further medical investigations such as genetic analysis of the tumor tissue.

When trained, the connection weights between the processing elements contain information regarding the relationship between the patient data (input) and the probability value (output), which can be used to predict the probability value for a new subject patient on the basis of his/her patient data.

Each network node represents a simple calculation of the weighted sum of inputs from prior nodes and a non-linear output function. The combined calculation of the network nodes relates the inputs to the output(s).

Separate networks can be developed for each property measurement or groups of properties can be included in a single network. Preferably, different dimensions of the patient data are combined at the end of the algorithm.

Training estimates network weights that allow the network to calculate (an) output value(s) close to the measured output value(s). A supervised training method can be used in which the output data is used to direct the training of the network weights. The network weights are initialized with small random values or with the weights of a prior partially trained network. The training data inputs are applied to the network and the output values are calculated for each training sample. The network output values are compared to the measured output values. A backpropagation algorithm is applied to correct the weight values in directions that reduce the error between measured and calculated outputs. The process is iterated until no further reduction in error can be made or until a predefined prediction accuracy has been reached.

A cross-validation method can be employed to split the data into training and validation data sets. The training data set is used in the backpropagation training of the network weights. The validation data set is used to verify that the trained network generalizes to make good predictions. The best network weight set can be taken as the one that best predicts the outputs of the test data set. Similarly, varying the number of network hidden nodes and determining the network that performs best with the data sets optimizes the number of hidden nodes.

Forward prediction uses the trained network to calculate a probability value indicating the probability of a patient suffering from cancer caused by an NTRK oncogenic fusion. Patient data are inputted into the trained network. A feed forward calculation through the network is made to predict the output property value(s). The predicted measurements can be compared to (a) property target value(s) or tolerance(s). Since the method of the invention is based on historical data of property values, the prediction of property values using such method typically have an error approaching the error of the empirical data, so that the predictions are often just as accurate as verification experiments.

In a preferred embodiment of the present invention, the prediction model is or comprises a convolutional neural network (CNN).

A CNN is a class of deep neural networks, most commonly applied to analyzing visual imagery. A CNN comprises an input layer with input neurons, an output layer with at least one output neuron, as well as multiple hidden layers between the input layer and the output layer.

The hidden layers of a CNN typically consist of convolutional layers, ReLU (Rectified Linear Units) layer i.e. activation function, pooling layers, fully connected layers and normalization layers.

The nodes in the CNN input layer are organized into a set of "filters" (feature detectors), and the output of each set of filters is propagated to nodes in successive layers of the network. The computations for a CNN include applying the convolution mathematical operation to each filter to produce the output of that filter. Convolution is a specialized kind of mathematical operation performed by two functions to produce a third function that is a modified version of one of the two original functions. In convolutional network terminology, the first function to the convolution can be referred to as the input, while the second function can be referred to as the convolution kernel. The output may be referred to as the feature map. For example, the input to a convolution layer can be a multidimensional array of data that defines the various color components of an input image. The convolution kernel can be a multidimensional array of parameters, where the parameters are adapted by the training process for the neural network.

By analysis of the CNN, one can reveal patterns in the data which are not obvious and were used preferred (i.e., weighted more strongly) by the CNN while analyzing the training data. This explainable AI approach helps to generate trust in the performance of the prediction model.

The present invention can be used to
a) detect NTRK fusion events in other indications than in those being trained on (i.e., an algorithm trained on thyroid data sets is useful in lung cancer data sets)
b) detect NTRK fusion events involving other TRK family members (i.e., an algorithm trained on NTRK1, NTRK3 fusions is useful to predict also NTRK2 fusions)
c) detect NTRK fusion events involving other fusion partners (i.e., an algorithm trained on LMNA-fusion data sets is useful also in TPM3-fusion data sets)
d) discover novel fusion partners (i.e., an algorithm trained on known fusion events might predict a fusion in a new data set which is then confirmed via molecular assay to involve a not yet described fusion partner of a NTRK family member).

The prediction model generates a probability value indicating the probability of a patient suffering from cancer caused by an NTRK oncogenic fusion. The probability value can be outputted to a user and/or stored in a database. The probability value can be a real number in the range from 0 to 1, whereas a probability value of 0 means that it is impossible that the cancer is caused by an NTRK oncogenic fusion, and a probability value of 1 means that there is no doubt that the cancer is caused by an NTRK oncogenic fusion. The probability value can also be expressed by a percentage.

In a preferred embodiment of the present invention, the probability value is compared with a predefined threshold value. In the event the probability value is lower than the threshold value, the probability that the patient suffers from cancer caused by an NTRK oncogenic fusion is low; treating the patient with a Trk inhibitor is not indicated; further investigations are required in order to determine the cause of cancer. In the event the probability value equals the threshold value or is greater than the threshold value, it is reasonable to assume that the cancer is caused by an NTRK oncogenic fusion; the treatment of the patient with a Trk inhibitor can be indicated; further investigations to verify the assumption can be initiated (e.g. performing a genetic analysis of the tumor tissue).

The threshold value can be a value between 0.5 and 0.99999999999, e.g. 0.8 (80%) or 0.81 (81 %) or 0.82 (82% ) or 0.83 (83%) or 0.84 (84%) or 0.85 (85%) or 0.86 (86%) or 0.87 (87 %) or 0.88 (88%) or 0.89 (89%) or 0.9 (90%) or 0.91 (91 %) or 0.92 (92% ) or 0.93 (93%) or 0.94 (94%) or 0.95 (95%) or 0.96 (96%) or 0.97 (97 %) or 0.98 (98%) or 0.99 (99%) or any other value (percentage).

The prediction model can also perform a classification. On the basis of the input data, the at least one histopathological image is assigned to one of at least two classes, a first class and a second class. The first class comprises images showing tumor tissue wherein the tumor is caused by an NTRK oncogenic fusion. The second class comprises images which do not show tumor tissue caused by an NTRK oncogenic fusion. In this case, the probability value represents the class the respective histopathological image is assigned to.

In a further aspect, the present invention relates to the use of a Trk inhibitor in the manufacture of a medicament for treating cancer in a subject patient, wherein the subject patient who shall be treated is one for whom a probability value is determined which is equal to or greater than a predefined threshold value, the probability value indicating the probability of a patient suffering from cancer caused by an NTRK oncogenic fusion, the probability value being determined by means of a prediction model on the basis of patient data.

In a further aspect, the present invention relates to a Trk inhibitor for use in treating cancer in a subject patient, wherein the subject patient who shall be treated is one for whom a probability value is determined which is equal to or greater than a predefined threshold value, the probability value indicating the probability of a patient suffering from cancer caused by an NTRK oncogenic fusion, the probability value being determined by means of a prediction model on the basis of patient data.

In a further aspect, the present invention relates to a Trk inhibitor for use in a method of treatment of cancer in a subject patient, wherein the subject patient who shall be treated is one for whom a probability value is determined which is equal to or greater than a predefined threshold value, the probability value indicating the probability of a patient suffering from cancer caused by an NTRK oncogenic fusion, the probability value being determined by means of a prediction model on the basis of patient data.

In a further aspect, the present invention relates to the use of a Trk inhibitor for the treatment of cancer in a subject patient, wherein the subject patient who shall be treated is one for whom a probability value is determined which is equal to or greater than a predefined threshold value, the probability value indicating the probability of a patient suffering from cancer caused by an NTRK oncogenic fusion, the probability value being determined by means of a prediction model on the basis of patient data.

In a further aspect, the present invention relates to a method for identifying a subject patient disposed to respond favorably to a Trk inhibitor for treating cancer, wherein the method comprises the following steps:
- receiving patient data of the subject patient, the patient data comprising at least one histopathological image of tumor tissue of the subject patient;
- inputting the patient data into a prediction model trained via machine learning with a set of training data, the prediction model being configured for identifying within the patient data one or more characteristics of an NTRK oncogenic fusion;
- receiving as an output from the prediction model a probability value, the probability value indicating the probability of the subject patient suffering from cancer caused by an NTRK oncogenic fusion;
- comparing the probability value with a predefined threshold value;
whereas the probability value is equal to or greater than the predefined threshold value.

**In** a further aspect, the present invention relates to a method of treating cancer in a subject patient, the method comprising the steps:
- receiving patient data of the subject patient, the patient data comprising at least one histopathological image of tumor tissue of the subject patient;
- inputting the patient data into a prediction model trained via machine learning with a set of training data, the prediction model being configured for identifying within the patient data one or more characteristics of an NTRK oncogenic fusion;
- receiving as an output from the prediction model a probability value, the probability value indicating the probability of the subject patient suffering from cancer caused by an NTRK oncogenic fusion;
- comparing the probability value with a predefined threshold value whereas the probability value is equal to or greater than the predefined threshold value;
- administering a therapeutically effective amount of a Trk inhibitor.

**In** a further aspect, the present invention relates to a kit comprising:
- a medicament comprising a Trk inhibitor, and
- a computer program product, the computer program product comprising a non-transitory computer-readable storage medium comprising processor-executable instructions with which to perform an operation for identifying one or more candidate signs indicative of an NTRK oncogenic fusion within patient data associated with a subject patient, the operation comprising:
   - receiving patient data of a subject patient suffering from cancer, the patient data comprising at least one histopathological image of tumor tissue of the subject patient;
   - inputting the patient data into a prediction model trained via machine learning with a set of training data, the prediction model being configured for identifying within the patient data one or more characteristics of an NTRK oncogenic fusion;
   - receiving as an output from the prediction model a probability value, the probability value indicating the probability of the subject patient suffering from cancer caused by an NTRK oncogenic fusion;
   - comparing the probability value with a predefined threshold value whereas the probability value is equal to or greater than the predefined threshold value.

**In** a preferred embodiment, the Trk inhibitor is Sitravatinib, Belizatinib, Entrectinib or Larotrectinib.

Sitravatinib (MGCD-516) is a small-molecule multi-kinase inhibitor that targets MET, AXL, MER, as well as members of the vascular endothelial growth factor receptor (VEGFR), platelet- derived growth factor receptor (PDGFR), discoidin domain receptor tyrosine kinase 2 (DDR2), and Trk families.

Belizatinib (TSR-011) is an oral dual ALK and pan-Trk inhibitor.

Entrectinib (N-[5-(3,5-Difluorobenzyl)-1H-indazol-3-yl]-4-(4-methyl-1-piperazinyl)-2-(tetrahydro-2H-pyran-4-ylamino)benzamide) is a an oral ATP-competitive, pan-Trk, ROS1, and ALK Trk inhibitor.

Larotrectinib ((3S)-N-{5-[(2R)-2-(2,5-difluorophenyl)pyrrolidin-1-yl]pyrazolo[1,5-a]pyrimidin-3-yl}-3-hydroxypyrrolidine-1-carboxamide) is an orally administered selective tropomyosin receptor kinases (Trk) inhibitor which can be used for the treatment of genomically defined cancers harbouring NTRK gene fusions including solid tumours, non-Hodgkin lymphoma, histiocytic disorders and primary CNS cancers. Preclinical development is underway for precursor cell lymphoblastic leukaemia-lymphoma (acute lymphoblastic leukaemia) in the US.

In a more preferred embodiment, the Trk inhibitor is Larotrectinib.

In a preferred embodiment of the present invention, the cancer is selected from: lung cancer, colorectal cancer, papillary thyroid cancer, glioblastoma multiforme, sarcoma, secretory breast cancer, mammary analog secretory carcinoma, non-Hodgkin lymphoma, histiocytic disorders and primary CNS (central nervous system) cancer.

In a further aspect, the present invention relates to a system comprising
- an input unit,
- a processing unit and
- an output unit,

wherein the input unit is configured to accept patient data of a subject patient, the patient data comprising at least one histopathological image of tumor tissue of the subject patient;
wherein the processing unit is configured to determine, on the basis of the patient data, a probability value, the probability value indicating the probability of the subject patient suffering from cancer caused by an NTRK oncogenic fusion;
wherein the processing unit is optionally configured to compare the probability value with a predefined threshold value and thereby determining a comparison result;
wherein the output unit is configured to display the probability value and/or to display the comparison result.

The operations in accordance with the teachings herein may be performed by at least one computer specially constructed for the desired purposes or general purpose computer specially configured for the desired purpose by at least one computer program stored in a typically non-transitory computer readable storage medium.

The term "non-transitory" is used herein to exclude transitory, propagating signals or waves, but to otherwise include any volatile or non-volatile computer memory technology suitable to the application.

The term "computer" should be broadly construed to cover any kind of electronic device with data processing capabilities, including, by way of non-limiting example, personal computers, servers, embedded cores, computing system, communication devices, processors (e.g. digital signal processor (DSP)), microcontrollers, field programmable gate array (FPGA), application specific integrated circuit (ASIC), etc.) and other electronic computing devices.

The term "process" as used above is intended to include any type of computation or manipulation or transformation of data represented as physical, e.g. electronic, phenomena which may occur or reside e.g. within registers and/or memories of at least one computer or processor. The term processor includes a single processing unit or a plurality of distributed or remote such units.

Any suitable processor/s, display and input means may be used to process, display e.g. on a computer screen or other computer output device, store, and accept information such as information used by or generated by any of the methods and system shown and described herein; the above processor/s, display and input means including computer programs, in accordance with some or all of the embodiments of the present invention. Any or all functionalities of the invention shown and described herein, such as but not limited to operations within flowcharts, may be performed by any one or more of: at least one conventional personal computer processor, workstation or other programmable device or computer or electronic computing device or processor, either general-purpose or specifically constructed, used for processing; a computer display screen and/or printer and/or speaker for displaying; machine-readable memory such as optical disks, CDROMs, DVDs, BluRays, magnetic-optical discs or other discs; RAMs, ROMs, EPROMs, EEPROMs, magnetic or optical or other cards, for storing, and keyboard or mouse for accepting. Modules shown and described herein may include any one or combination or plurality of: a server, a data processor, a memory / computer storage, a communication interface, a computer program stored in memory/computer storage.

Any suitable input device, such as but not limited to a camera sensor, may be used to generate or otherwise provide information received by the system and methods shown and described herein. Any suitable output device or display may be used to display or output information generated by the system and methods shown and described herein. Any suitable processor/s may be employed to compute or generate information as described herein and/or to perform functionalities described herein and/or to implement any engine, interface or other system described herein. Any suitable computerized data storage e.g. computer memory may be used to store information received by or generated by the systems shown and described herein. Functionalities shown and described herein may be divided between a server computer and a plurality of client computers. These or any other computerized components shown and described herein may communicate between themselves via a suitable computer network.

A system which is particularly suitable for executing the operation / steps / methods of the presentation invention is disclosed in WO2018/184194A.

Some implementations of the present disclosure will now be described more fully hereinafter with reference to the accompanying drawings, in which some, but not all implementations of the disclosure are shown. Indeed, various implementations of the disclosure may be embodied in many different forms and should not be construed as limited to the implementations set forth herein; rather, these example implementations are provided so that this disclosure will be thorough and complete, and will fully convey the scope of the disclosure to those skilled in the art. As used herein, for example, the singular forms "a", "an", "the" and the like include plural referents unless the context clearly dictates otherwise. The terms "data", "information", "content" and similar terms may be used interchangeably, according to some example implementations of the present invention, to refer to data capable of being transmitted, received, operated on, and/or stored. Also, for example, reference may be made herein to quantitative measures, values, relationships or the like. Unless otherwise stated, any one or more if not all of these may be absolute or approximate to account for acceptable variations that may occur, such as those due to engineering tolerances or the like. Like reference numerals refer to like elements throughout.

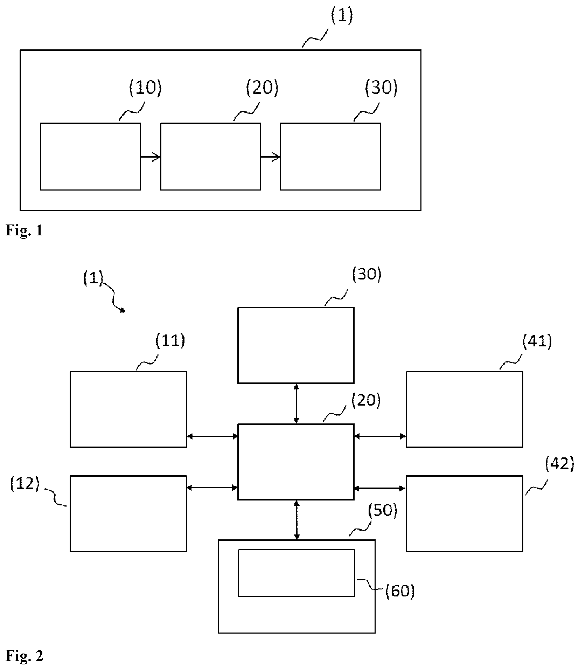

Fig. 1 shows schematically one embodiment of the system (1) according to the present invention. It comprises an input unit (10), a processing unit (20) and an output unit (30). The input unit (10) accepts patient data and forwards them to the processing unit (20) (depicted by the arrow between the input unit and the processing unit). The processing unit (20) determines a probability value on the basis of the patient data. The processing unit is optionally configured to compare the probability value with a predefined threshold value and thereby determining a comparison result. The probability value and/or the comparison result is/are transmitted from the processing unit (20) to the output unit (30) (depicted by the arrow between the processing unit and the output unit). The output unit (30) displays the probability value and/or the comparison result. The input unit (10), the processing unit (20) and the output unit (30) can be components of one or more computer systems.

Fig. 2 illustrates a computer system (1) according to some example implementations of the present disclosure in more detail. Generally, a computer system of exemplary implementations of the present disclosure may be referred to as a computer and may comprise, include, or be embodied in one or more fixed or portable electronic devices. The computer may include one or more of each of a number of components such as, for example, processing unit (20) connected to a memory (50) (e.g., storage device).

The processing unit (20) may be composed of one or more processors alone or in combination with one or more memories. The processing unit is generally any piece of computer hardware that is capable of processing information such as, for example, data, computer programs and/or other suitable electronic information. The processing unit is composed of a collection of electronic circuits some of which may be packaged as an integrated circuit or multiple interconnected integrated circuits (an integrated circuit at times more commonly referred to as a "chip"). The processing unit may be configured to execute computer programs, which may be stored onboard the processing unit or otherwise stored in the memory (50) of the same or another computer.

The processing unit (20) may be a number of processors, a multi-core processor or some other type of processor, depending on the particular implementation. Further, the processing unit may be implemented using a number of heterogeneous processor systems in which a main processor is present with one or more secondary processors on a single chip. As another illustrative example, the processing unit may be a symmetric multi-processor system containing multiple processors of the same type. **In** yet another example, the processing unit may be embodied as or otherwise include one or more ASICs, FPGAs or the like. Thus, although the processing unit may be capable of executing a computer program to perform one or more functions, the processing unit of various examples may be capable of performing one or more functions without the aid of a computer program. **In** either instance, the processing unit may be appropriately programmed to perform functions or operations according to example implementations of the present disclosure.

The memory (50) is generally any piece of computer hardware that is capable of storing information such as, for example, data, computer programs (e.g., computer-readable program code (60)) and/or other suitable information either on a temporary basis and/or a permanent basis. The memory may include volatile and/or non-volatile memory, and may be fixed or removable. Examples of suitable memory include random access memory (RAM), read-only memory (ROM), a hard drive, a flash memory, a thumb drive, a removable computer diskette, an optical disk, a magnetic tape or some combination of the above. Optical disks may include compact disk - read only memory (CD-ROM), compact disk - read/write (CD-R/W), DVD, Blu-ray disk or the like. In various instances, the memory may be referred to as a computer-readable storage medium. The computer-readable storage medium is a non-transitory device capable of storing information, and is distinguishable from computer-readable transmission media such as electronic transitory signals capable of carrying information from one location to another. Computer-readable medium as described herein may generally refer to a computer-readable storage medium or computer-readable transmission medium.

In addition to the memory (50), the processing unit (20) may also be connected to one or more interfaces for displaying, transmitting and/or receiving information. The interfaces may include one or more communications interfaces and/or one or more user interfaces. The communications interface(s) may be configured to transmit and/or receive information, such as to and/or from other computer(s), network(s), database(s) or the like. The communications interface may be configured to transmit and/or receive information by physical (wired) and/or wireless communications links. The communications interface(s) may include interface(s) (41) to connect to a network, such as using technologies such as cellular telephone, Wi-Fi, satellite, cable, digital subscriber line (DSL), fiber optics and the like. In some examples, the communications interface(s) may include one or more short-range communications interfaces (42) configured to connect devices using short-range communications technologies such as NFC, RFID, Bluetooth, Bluetooth LE, ZigBee, infrared (e.g., IrDA) or the like.

The user interfaces may include a display (30). The display may be configured to present or otherwise display information to a user, suitable examples of which include a liquid crystal display (LCD), light-emitting diode display (LED), plasma display panel (PDP) or the like. The user input interface(s) (11) may be wired or wireless, and may be configured to receive information from a user into the computer system (1), such as for processing, storage and/or display. Suitable examples of user input interfaces include a microphone, image or video capture device, keyboard or keypad, joystick, touch-sensitive surface (separate from or integrated into a touchscreen) or the like. In some examples, the user interfaces may include automatic identification and data capture (AIDC) technology (12) for machine-readable information. This may include barcode, radio frequency identification (RFID), magnetic stripes, optical character recognition (OCR), integrated circuit card (ICC), and the like. The user interfaces may further include one or more interfaces for communicating with peripherals such as printers and the like.

As indicated above, program code instructions may be stored in memory, and executed by processing unit that is thereby programmed, to implement functions of the systems, subsystems, tools and their respective elements described herein. As will be appreciated, any suitable program code instructions may be loaded onto a computer or other programmable apparatus from a computer-readable storage medium to produce a particular machine, such that the particular machine becomes a means for implementing the functions specified herein. These program code instructions may also be stored in a computer-readable storage medium that can direct a computer, processing unit or other programmable apparatus to function in a particular manner to thereby generate a particular machine or particular article of manufacture. The instructions stored in the computer-readable storage medium may produce an article of manufacture, where the article of manufacture becomes a means for implementing functions described herein. The program code instructions may be retrieved from a computer-readable storage medium and loaded into a computer, processing unit or other programmable apparatus to configure the computer, processing unit or other programmable apparatus to execute operations to be performed on or by the computer, processing unit or other programmable apparatus.

Retrieval, loading and execution of the program code instructions may be performed sequentially such that one instruction is retrieved, loaded and executed at a time. In some example implementations, retrieval, loading and/or execution may be performed in parallel such that multiple instructions are retrieved, loaded, and/or executed together. Execution of the program code instructions may produce a computer-implemented process such that the instructions executed by the computer, processing circuitry or other programmable apparatus provide operations for implementing functions described herein.

Execution of instructions by processing unit, or storage of instructions in a computer-readable storage medium, supports combinations of operations for performing the specified functions. In this manner, a computer system (1) may include processing unit (20) and a computer-readable storage medium or memory (50) coupled to the processing circuitry, where the processing circuitry is configured to execute computer-readable program code (60) stored in the memory. It will also be understood that one or more functions, and combinations of functions, may be implemented by special purpose hardware-based computer systems and/or processing circuitry which perform the specified functions, or combinations of special purpose hardware and program code instructions.

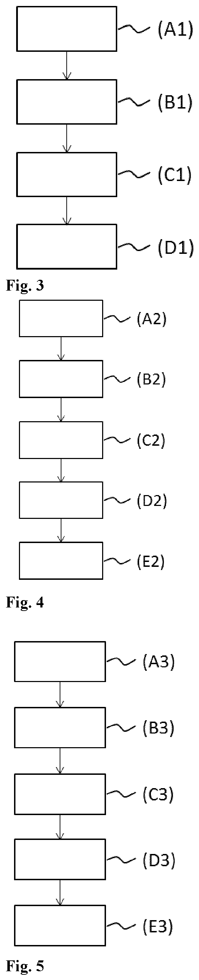

Fig. 3 shows schematically in the form of a flow chart the steps which are performed according to some embodiments of the present invention.

The steps are:
(A1) receiving patient data of a subject patient suffering from cancer, the patient data comprising at least one histopathological image of tumor tissue of the subject patient;
(B1) inputting the patient data into a prediction model trained via machine learning with a set of training data;
(C1) obtaining from the prediction model a probability value, the probability value indicating the probability of the subject patient suffering from cancer caused by an NTRK oncogenic fusion;
(D1) outputting and/or storing the probability value.

Fig. 4 shows schematically in the form of a flow chart the steps which are performed according to some embodiments of the present invention.

The steps are:
(A2) receiving patient data of a subject patient suffering from cancer, the patient data comprising at least one histopathological image of tumor tissue of the subject patient;
(B2) inputting the patient data into a prediction model trained via machine learning with a set of training data;
(C2) obtaining from the prediction model a probability value, the probability value indicating the probability of the subject patient suffering from cancer caused by an NTRK oncogenic fusion;
(D2) comparing the probability value with a predefined threshold value and thereby determining a comparison result;
(E2) outputting and/or storing the probability value and/or the comparison result.

Fig. 5 shows schematically in the form of a flow chart the steps which are performed according to some embodiments of the present invention.

The steps are:
(A3) receiving patient data of a subject patient suffering from cancer, the patient data comprising at least one histopathological image of tumor tissue of the subject patient;
(B3) inputting the patient data into a prediction model trained via machine learning with a set of training data;
(C3) obtaining from the prediction model a probability value, the probability value indicating the probability of the subject patient suffering from cancer caused by an NTRK oncogenic fusion;
(D3) comparing the probability value with a predefined threshold value;
(E3) in the event that the probability value is equal to or greater than the threshold value: initiating further investigations for verification of the indication that the subject patient suffers from cancer caused by an NTRK oncogenic fusion or administering a therapeutically effect amount of a Trk inhibitor.

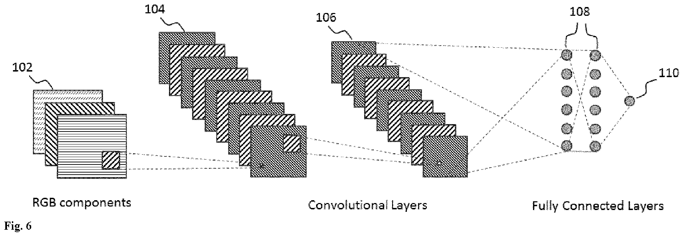

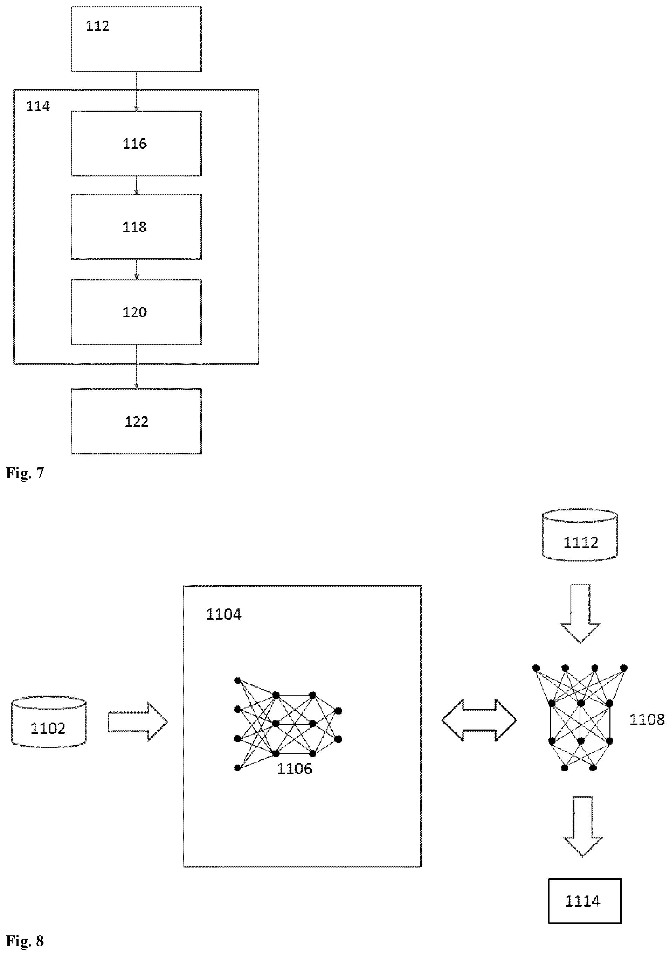

Figs. 6 and 7 illustrate schematically an exemplary convolutional neural network which can be used to determine a probability value according to the present invention. Fig. 6 illustrates various layers within a CNN. As shown in Fig. 6, an exemplary CNN used to identify within patient data one or more characteristics of an NTRK oncogenic fusion can receive input (102) describing the red, green, and blue (RGB) components of an input histopathological image. The input (102) can be processed by multiple convolutional layers (e.g., convolutional layer (104), convolutional layer (106)). The output from the multiple convolutional layers may optionally be processed by a set of fully connected layers (108, 110). Neurons in a fully connected layer have full connections to all activations in the previous layer. The output from the fully connected layers (108) can be used to generate an output result (110) from the network. The output result can be the probability value indicating the probability of a patient suffering from cancer caused by an NTRK oncogenic fusion.

The activations within the fully connected layers (108) can be computed using matrix multiplication instead of convolution. Not all CNN implementations make use of fully connected layers. For example, in some implementations, the convolutional layer (106) can generate output for the CNN.

The convolutional layers are sparsely connected, which differs from traditional neural network configuration found in the fully connected layers (108). Traditional neural network layers are fully connected, such that every output unit interacts with every input unit. However, the convolutional layers are sparsely connected because the output of the convolution of a field is input (instead of the respective state value of each of the nodes in the field) to the nodes of the subsequent layer, as illustrated. The kernels associated with the convolutional layers perform convolution operations, the output of which is sent to the next layer. The dimensionality reduction performed within the convolutional layers is one aspect that enables the CNN to process large images.

Fig. 7 illustrates exemplary computation stages within a convolutional layer of a CNN. Input (112) to a convolutional layer (114) of a CNN can be processed in three stages of the convolutional layer (114). The three stages can include a convolution stage (116), a detector stage (118), and a pooling stage (120). The convolution layer (114) can then output data to a successive convolutional layer. The final convolutional layer of the network can generate output feature map data or provide input to a fully connected layer, for example, to generate a classification or regression value.

In the convolution stage (116), the convolutional layer (114) can perform several convolutions in parallel to produce a set of linear activations. The convolution stage (116) can include an affine transformation, which is any transformation that can be specified as a linear transformation plus a translation. Affine transformations include rotations, translations, scaling, and combinations of these transformations. The convolution stage computes the output of functions (e.g., neurons) that are connected to specific regions in the input, which can be determined as the local region associated with the neuron. The neurons compute a dot product between the weights of the neurons and the region in the local input to which the neurons are connected. The output from the convolution stage (116) defines a set of linear activations that are processed by successive stages of the convolutional layer (114).

The linear activations can be processed by a detector stage (118). In the detector stage (118), each linear activation is processed by a non-linear activation function. The non-linear activation function increases the nonlinear properties of the overall network without affecting the receptive fields of the convolution layer. Several types of non-linear activation functions may be used. One particular type is the rectified linear unit (ReLU), which uses an activation function defined as *f(x) = max(0, x),* such that the activation is threshold at zero.

The pooling stage (120) uses a pooling function that replaces the output of the convolutional layer (106) with a summary statistic of the nearby outputs. The pooling function can be used to introduce translation invariance into the neural network, such that small translations to the input do not change the pooled outputs. Invariance to local translation can be useful in scenarios where the presence of a feature in the input data is more important than the precise location of the feature. Various types of pooling functions can be used during the pooling stage (120), including max pooling, average pooling, and 12-norm pooling. Additionally, some CNN implementations do not include a pooling stage. Instead, such implementations substitute and additional convolution stage having an increased stride relative to previous convolution stages.

The output from the convolutional layer (114) can then be processed by the next layer (122). The next layer (122) can be an additional convolutional layer or one of the fully connected layers (108). For example, the first convolutional layer (104) of Fig. 6 can output to the second convolutional layer (106), while the second convolutional layer can output to a first layer of the fully connected layers (108).

Fig. 8 illustrates an exemplary training and deployment of a neural network. Once a given network has been structured for a task the neural network is trained using a training dataset (1102).

To start the training process the initial weights may be chosen randomly or by pre-training using a deep belief network. The training cycle then be performed in either a supervised or unsupervised manner. Supervised learning is a learning method in which training is performed as a mediated operation, such as when the training dataset (1102) includes input paired with the desired output for the input, or where the training dataset includes input having known output and the output of the neural network is manually graded. The network processes the inputs and compares the resulting outputs against a set of expected or desired outputs. Errors are then propagated back through the system. The training framework (1104) can adjust the weights that control the untrained neural network (1106). The training framework (1104) can provide tools to monitor how well the untrained neural network (1106) is converging towards a model suitable to generating correct answers based on known input data. The training process occurs repeatedly as the weights of the network are adjusted to refine the output generated by the neural network. The training process can continue until the neural network reaches a statistically desired accuracy associated with a trained neural network (1108). The trained neural network (1108) can then be deployed to implement any number of machine learning operations. A new set of patient data (1112) can be inputted into the trained neural network (1108) to determine a probability value, the probability value indicating the probability of a patient suffering from cancer caused by a mutation of a neurotrophic receptor tyrosine kinase (NTRK) gene.

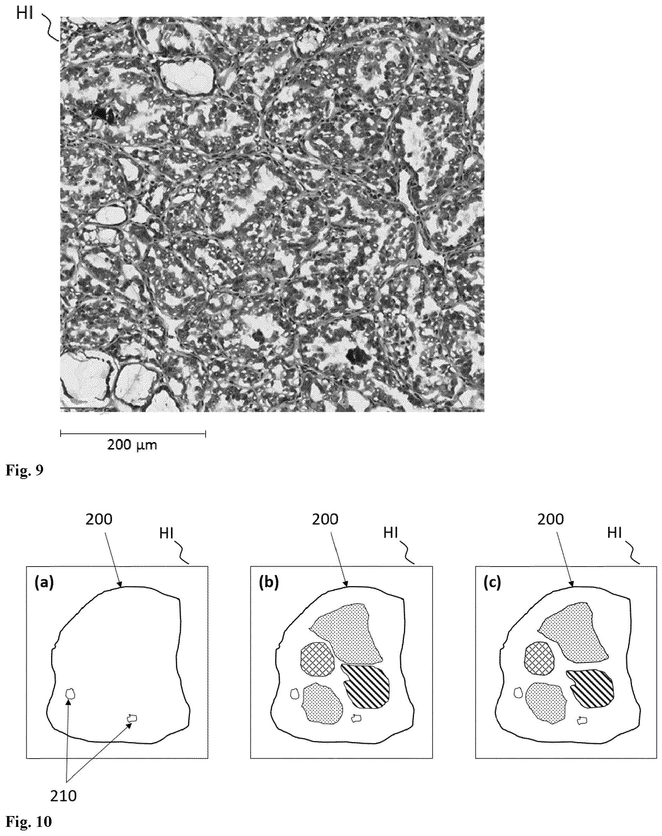

Fig. 9 is an example of a histopathological image (HI) of tumor tissue of a patient suffering from cancer.

Fig. 10 is a schematic draw which illustrates of the performance of the prediction model and an example of the way to represent its output.

Fig. 10 (a) illustrates a digital image of an HE stained, formalin-fixed, paraffin-embedded (FFPE) tumor tissue section. The tissue (200) contains holes (210). Such an image serves as input to the prediction model. Note that the details of the tissue revealed by the HE stain (which result in different colors and intensities with in the tissue section) are omitted for clarity. However, these reveal to the trained person only the position, shape, and thus type of cells, not their molecular composition.

Fig. 10 (b) illustrates an output example of the prediction tool. A digital image is generated corresponding to the layout of the tissue scan in Fig. 10 (a). The tissue scan is usually overlaid by a pseudo-colored image in which the color or the intensity of a color corresponds to the probability that an area of the tissue shows a tumor cancer caused by an NTRK oncogenic fusion (here only black/white images are shown). In a preferred embodiment, the colors are chosen to represent the typical colors (eg brownish or redish) of immunohistochemistry dyes to generate a 'pseudo IHC image'. Colors are indicated in the figure by areas which are dotted (indicating a predicted weak staining intensity), cross hatched (moderately stained), or thickly hatched (strongly stained).

Fig. 10 (c) illustrates a digital image of an adjacent FFPE tumor tissue section stained with an antibody for indicating presence of TRK proteins using immunohistochemistry. Note that the digital image corresponds very well to the predictions made by the prediction model as shown in Fig. 10 (b), even though the molecular assay was conducted on an adjacent tissue section.

Note that this performance holds true at higher magnification as well, when zooming in from the tissue to the morphology / cellular level. In a preferred embodiment, predicted images such as shown in Fig. 10 (b) turn from HE colors to pseudo IHC colors in (almost) real time when zooming in to one zone or moving across the tissue. This is enabled by edge analysis, and enables the system according to the present invention to process information locally and respond more quickly to situations. This feature also allows our tool to work in telepathology settings, even in mobile computing devices such as smartphones. In a preferred embodiment, the prediction model is embedded in a website for convenient installation on routine computer devices.

IHC can be performed for example with the research use only panTRK monoclonal antibody EPR17341 (Abcam, Cambridge, MA), see Hechtman et al., The American Journal of Surgical Pathology (2017), 41 (11), 1547 - 1551, PMID 28719467, using for example a Ventana Autostainer Discovery Ultra equipment.

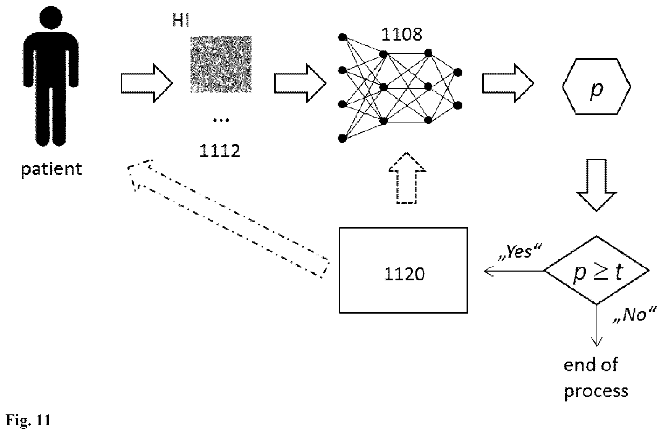

Fig. 11 shows schematically an embodiment of a process for using the present invention in practice. Starting point of the process is a patient. The patient is suffering from cancer. One aim of the process is to find out whether the cancer is caused by a mutation of a neurotrophic receptor tyrosine kinase (NTRK) gene. In a first step, patient data (1112) are collected/retrieved. The patient data (1112) comprise at least one histopathological image (HI) of tumor tissue of the patient. In a following step, the patient data are inputted into a prediction model (1108) which is trained by supervised learning. For the supervised training, patient data of a multitude of patients can be used for whom it has been determined whether they suffer from cancer caused by a mutation of a neurotrophic receptor tyrosine kinase (NTRK) gene or whether they suffer from cancer not caused by a mutation of a neurotrophic receptor tyrosine kinase (NTRK) gene.

The trained prediction model (1108) is configured to determine a probability value p from the inputted patient data (1112). The probability value indicates the probability of the patient suffering from cancer caused by a mutation of a neurotrophic receptor tyrosine kinase (NTRK) gene.

In a further step, the probability value *p* is compared with a predefined threshold value *t.*

If the probability value *p* is smaller than the predefined threshold value *t (p* ≥ *t: "No"),* then it is unlikely that the patient suffers from cancer caused by a mutation of a neurotrophic receptor tyrosine kinase (NTRK) gene; other causes for the cancer have to be identified; the process ends.

If the probability value *p* is equal to or greater than the predefined threshold value *t (p* ≥ *t: "Yes"),* then it is likely that the patient suffers from cancer caused by a mutation of a neurotrophic receptor tyrosine kinase (NTRK) gene. A subsequent step (1120) is initiated. In one embodiment of the present invention, further investigations are initiated in order to verify that the cancer is caused by a mutation of a neurotrophic receptor tyrosine kinase (NTRK) gene. This can be done e.g. by a genetic analysis of the tumor tissue. The results of the further investigations can be used to optimize (further supervised learning) the prediction model (1108). If the further investigation verifies that the cancer is caused by a mutation of a neurotrophic receptor tyrosine kinase (NTRK) gene, the patient can be treated with a Trk inhibitor. In another embodiment, the likelihood of the cancer being caused by a mutation of a neurotrophic receptor tyrosine kinase (NTRK) gene is considered to be so high that the patient is treated directly (without any further genetic analysis of the tumor tissue) with a Trk inhibitor.

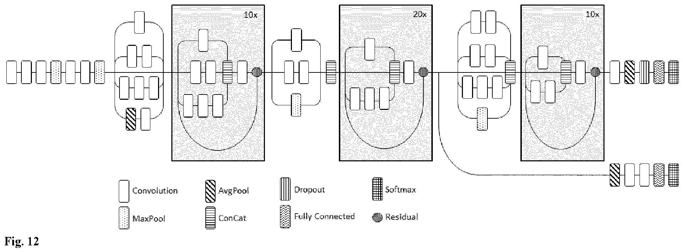

Figs. 12 to 14 illustrate an example of the present invention in which an artificial neural network was set up for the prediction of probability values, each probability value indicating the probability of a subject patient suffering from cancer of the thyroid gland caused by an NTRK oncogenic fusion.

Fig. 12 shows the layer structure of the artificial neural network. The network is provided by IBM under the name Inception ResNet v2 (https://developer.ibm.com/exchanges/models/all/max-inception-resnet-v2/). The network pre-trained on ImageNet was used with transfer learning (in Keras+Tensorflow).

The data sets for training and verification were taken from The Cancer Genome Atlas (TCGA: https://portal.gdc.cancer.gov/). Further data were obtained from Auria, the biobank of Turku University Hospital, Finland.

Digital images in .svs format of HE stained tissues sections (cryo and or FFPE formalin fixed paraffin embedded) were used as histopathological images. The age of the patient at diagnosis was also used as input parameter for prediction. Information extracted from the whole exome sequencing data available TCGA on the mutational status of key oncogenes were used as well as NGS panel results (Illumina TST 170) on the Auria samples.

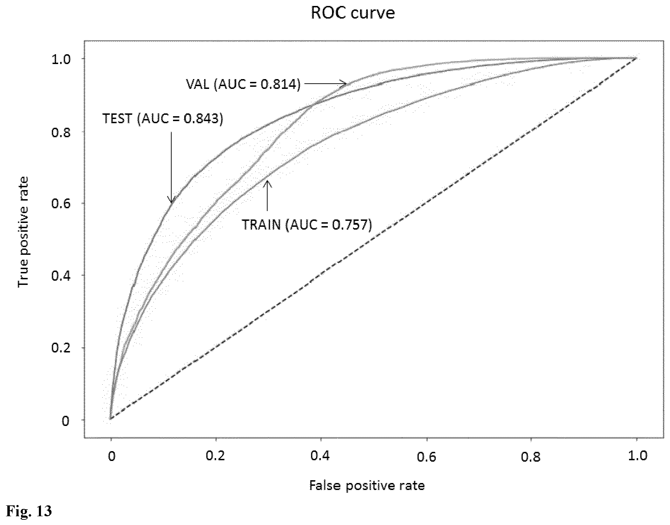

Fig. 13 shows the results in the form of a receiver operating characteristic curve (ROC curve). TRAIN refers to the training data set, VAL refers to the validation data set and TEST refers to the testing data set. The training data set was used to train the prediction model, the validation data set was used to validate the prediction model and the testing data set was used for prediction purposes. AUC means area under the curve.

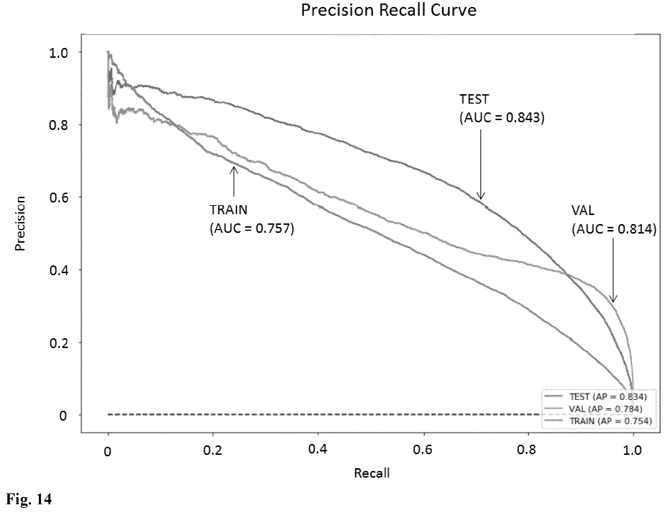

Fig. 14 shows the result in the form of a precision recall curve. TRAIN refers to the training data set, VAL refers to the validation data set and TEST refers to the testing data set. The training data set was used to train the prediction model, the validation data set was used to validate the prediction model and the testing data set was used for prediction purposes. AUC means area under the curve.

## Claims

1. A computer-implemented method for identifying one or more candidate signs indicative of an NTRK oncogenic fusion within patient data associated with a subject patient, the method comprising:
- receiving patient data of a subject patient suffering from cancer, the patient data comprising at least one histopathological image of tumor tissue of the subject patient;
- inputting the patient data into a prediction model trained via machine learning with a set of training data, the training data comprising histopathological images, the prediction model being configured for identifying within the patient data one or more characteristics of an NTRK oncogenic fusion;
- receiving as an output from the prediction model a probability value, the probability value indicating the probability of the subject patient suffering from cancer caused by an NTRK oncogenic fusion.

2. Method according to claim 1, further comprising the steps:
- comparing the probability value with a predefined threshold value and thereby determining a comparison result;
- outputting and/or storing the probability value and/or the comparison result.

3. Method according to claim 1, further comprising the steps:
- comparing the probability value with a predefined threshold value;
- in the event that the probability value is equal to or greater than the threshold value: initiating further investigations for verification of the indication that the subject patient suffers from cancer caused by an NTRK oncogenic fusion.

4. Method for identifying one or more candidate signs indicative of an NTRK oncogenic fusion within patient data associated with a subject patient, the method comprising the steps:
- receiving an image from a histopathological tissue slide of a hematoxylin and eosin stained tumor tissue sample of a subject patient;
- removing, using threshold techniques, from the image background areas, thereby receiving an image consisting of areas in which tissue material is shown,
- dividing the image into tumor regions and healthy regions
- removing healthy regions from the images
- inputting the image of tiles of the image into a classification model
- receiving as an output from the classification model for the image or each tile of the image an information to which of a number of classes the image or the tile of the image belongs to, the number of classes comprising at least two classes, a first class and a second class, the first class indicating the presence of an NTRK fusion cancer in the image or tile of the image, the second class indicating the non-presence of an NTRK fusion cancer in the image or tile of the image.

5. Method according to claim 4, further comprising the steps:
- coloring areas in the image or in the tiles of the image in a pre-defined color for which the presence of an NTRK fusion cancer was determined.
- outputting the colored image.

6. A system, comprising:
a processor; and
a memory storing an application program configured to perform, when executed by the processor, an operation for identifying one or more candidate signs indicative of an NTRK oncogenic fusion within patient data associated with a subject patient, the operation comprising:
- receiving patient data of a subject patient suffering from cancer, the patient data comprising at least one histopathological image of tumor tissue of the subject patient;
- inputting the patient data into a prediction model trained via machine learning with a set of training data, the training data comprising histopathological images, the prediction model being configured for identifying within the patient data one or more characteristics of an NTRK oncogenic fusion;
- receiving as an output from the prediction model a probability value, the probability value indicating the probability of the subject patient suffering from cancer caused by an NTRK oncogenic fusion.

7. A non-transitory computer-readable storage medium comprising processor-executable instructions with which to perform an operation for identifying one or more candidate signs indicative of an NTRK oncogenic fusion within patient data associated with a subject patient, the operation comprising:
- receiving patient data of a subject patient suffering from cancer, the patient data comprising at least one histopathological image of tumor tissue of the subject patient;
- inputting the patient data into a prediction model trained via machine learning with a set of training data, the training data comprising histopathological images, the prediction model being configured for identifying within the patient data one or more characteristics of an NTRK oncogenic fusion;
- receiving as an output from the prediction model a probability value, the probability value indicating the probability of the subject patient suffering from cancer caused by an NTRK oncogenic fusion.

## Patentansprüche

1. Computerimplementiertes Verfahren zum Identifizieren eines oder mehrerer Kandidatenzeichen, die auf eine onkogene NTRK-Fusion hinweisen, innerhalb von Patientendaten, die einem Subjektpatienten zugeordnet sind, wobei das Verfahren umfasst:
• Empfangen von Patientendaten eines an Krebs leidenden Subjektpatienten, wobei die Patientendaten mindestens ein histopathologisches Bild von Tumorgewebe des Subjektpatienten umfassen;
• Eingeben der Patientendaten in ein Vorhersagemodell, das mittels maschinellen Lernens mit einem Satz von Trainingsdaten trainiert wurde, wobei die Trainingsdaten histopathologische Bilder umfassen, wobei das Vorhersagemodell dazu konfiguriert ist, innerhalb der Patientendaten ein oder mehrere Merkmale einer onkogenen NTRK-Fusion zu identifizieren;
• Empfangen eines Wahrscheinlichkeitswerts als Ausgabe von dem Vorhersagemodell, wobei der Wahrscheinlichkeitswert die Wahrscheinlichkeit angibt, dass der Subjektpatient an Krebs leidet, der durch eine onkogene NTRK-Fusion verursacht wird.

2. Verfahren nach Anspruch 1, ferner umfassend die Schritte:
• Vergleichen des Wahrscheinlichkeitswerts mit einem vordefinierten Schwellenwert und dadurch Bestimmen eines Vergleichsergebnisses;
• Ausgeben und/oder Speichern des Wahrscheinlichkeitswerts und/oder des Vergleichsergebnisses.

3. Verfahren nach Anspruch 1, ferner umfassend die Schritte:
• Vergleichen des Wahrscheinlichkeitswerts mit einem vordefinierten Schwellenwert;
• für den Fall, dass der Wahrscheinlichkeitswert gleich dem Schwellenwert oder größer als der Schwellenwert ist: Einleiten weiterer Untersuchungen zur Verifizierung des Hinweises, dass der Subjektpatient an Krebs leidet, der durch eine onkogene NTRK-Fusion verursacht wird.

4. Verfahren zum Identifizieren eines oder mehrerer Kandidatenzeichen, die auf eine onkogene NTRK-Fusion hinweisen, innerhalb von Patientendaten, die einem Subjektpatienten zugeordnet sind, wobei das Verfahren die folgenden Schritte umfasst:
• Empfangen eines Bildes von einem histopathologischen Gewebeschnitt einer mit Hämatoxylin und Eosin gefärbten Tumorgewebeprobe eines Subjektpatienten;
• Entfernen von Hintergrundbereichen aus dem Bild unter Verwendung von Schwellenwerttechniken, wobei dadurch ein Bild erhalten wird, das aus Bereichen besteht, in denen Gewebematerial gezeigt wird;
• Unterteilen des Bildes in Tumorbereiche und gesunde Bereiche;
• Entfernen der gesunden Bereiche aus den Bildern;
• Eingeben des Bildes oder von Kacheln des Bildes in ein Klassifikationsmodell;
• Empfangen einer Information als Ausgabe von dem Klassifikationsmodell für das Bild oder jede Kachel des Bildes, zu welcher einer Anzahl von Klassen das Bild oder die Kachel des Bildes gehört, wobei die Anzahl von Klassen mindestens zwei Klassen umfasst, eine erste Klasse und eine zweite Klasse, wobei die erste Klasse das Vorhandensein eines NTRK-Fusionskrebses in dem Bild oder der Kachel des Bildes angibt und die zweite Klasse das Nichtvorhandensein eines NTRK-Fusionskrebses in dem Bild oder der Kachel des Bildes angibt.

5. Verfahren nach Anspruch 4, ferner umfassend die Schritte:
• Einfärben von Bereichen in dem Bild oder in den Kacheln des Bildes in einer vordefinierten Farbe, für die das Vorhandensein eines NTRK-Fusionskrebses bestimmt wurde;
• Ausgeben des eingefärbten Bildes.

6. System, umfassend:
einen Prozessor; und
einen Speicher, der ein Anwendungsprogramm speichert, das dazu konfiguriert ist, bei Ausführung durch den Prozessor eine Operation zum Identifizieren eines oder mehrerer Kandidatenzeichen durchzuführen, die auf eine onkogene NTRK-Fusion hinweisen, innerhalb von Patientendaten, die einem Subjektpatienten zugeordnet sind, wobei die Operation umfasst:
• Empfangen von Patientendaten eines an Krebs leidenden Subjektpatienten, wobei die Patientendaten mindestens ein histopathologisches Bild von Tumorgewebe des Subjektpatienten umfassen;
• Eingeben der Patientendaten in ein Vorhersagemodell, das mittels maschinellen Lernens mit einem Satz von Trainingsdaten trainiert wurde, wobei die Trainingsdaten histopathologische Bilder umfassen, wobei das Vorhersagemodell dazu konfiguriert ist, innerhalb der Patientendaten ein oder mehrere Merkmale einer onkogenen NTRK-Fusion zu identifizieren;
• Empfangen eines Wahrscheinlichkeitswerts als Ausgabe von dem Vorhersagemodell, wobei der Wahrscheinlichkeitswert die Wahrscheinlichkeit angibt, dass der Subjektpatient an Krebs leidet, der durch eine onkogene NTRK-Fusion verursacht wird.

7. Nichtflüchtiges computerlesbares Speichermedium, umfassend prozessorausführbare Anweisungen, mit denen eine Operation zum Identifizieren eines oder mehrerer Kandidatenzeichen durchführbar ist, die auf eine onkogene NTRK-Fusion hinweisen, innerhalb von Patientendaten, die einem Subjektpatienten zugeordnet sind, wobei die Operation umfasst:
• Empfangen von Patientendaten eines an Krebs leidenden Subjektpatienten, wobei die Patientendaten mindestens ein histopathologisches Bild von Tumorgewebe des Subjektpatienten umfassen;
• Eingeben der Patientendaten in ein Vorhersagemodell, das mittels maschinellen Lernens mit einem Satz von Trainingsdaten trainiert wurde, wobei die Trainingsdaten histopathologische Bilder umfassen, wobei das Vorhersagemodell dazu konfiguriert ist, innerhalb der Patientendaten ein oder mehrere Merkmale einer onkogenen NTRK-Fusion zu identifizieren;
• Empfangen eines Wahrscheinlichkeitswerts als Ausgabe von dem Vorhersagemodell, wobei der Wahrscheinlichkeitswert die Wahrscheinlichkeit angibt, dass der Subjektpatient an Krebs leidet, der durch eine onkogene NTRK-Fusion verursacht wird.

## Revendications

1. Procédé mis en œuvre par ordinateur pour identifier un ou plusieurs signes candidats indiquant une fusion oncogène NTRK dans des données patient associées à un patient sujet, le procédé comprenant :
• la réception de données patient d'un patient sujet souffrant d'un cancer, les données patient comprenant au moins une image histopathologique de tissu tumoral du patient sujet ;
• l'introduction des données patient dans un modèle de prédiction entraîné par apprentissage automatique avec un ensemble de données d'entraînement, les données d'entraînement comprenant des images histopathologiques, le modèle de prédiction étant configuré pour identifier dans les données patient une ou plusieurs caractéristiques d'une fusion oncogène NTRK ;
• la réception en sortie du modèle de prédiction d'une valeur de probabilité, la valeur de probabilité indiquant la probabilité que le patient sujet souffre d'un cancer causé par une fusion oncogène NTRK.

2. Procédé selon la revendication 1, comprenant en outre les étapes suivantes :
• la comparaison de la valeur de probabilité avec une valeur seuil prédéfinie et la détermination ainsi d'un résultat de comparaison ;
• la sortie et/ou le stockage de la valeur de probabilité et/ou du résultat de comparaison.

3. Procédé selon la revendication 1, comprenant en outre les étapes suivantes :
• la comparaison de la valeur de probabilité avec une valeur seuil prédéfinie ;
• dans le cas où la valeur de probabilité est égale à la valeur seuil ou supérieure à la valeur seuil : l'initiation d'investigations supplémentaires pour la vérification de l'indication que le patient sujet souffre d'un cancer causé par une fusion oncogène NTRK.

4. Procédé pour identifier un ou plusieurs signes candidats indiquant une fusion oncogène NTRK dans des données patient associées à un patient sujet, le procédé comprenant les étapes suivantes :
• la réception d'une image provenant d'une lame histopathologique de tissu d'un échantillon de tissu tumoral coloré à l'hématoxyline et à l'éosine d'un patient sujet ;
• l'élimination, à l'aide de techniques de seuillage, des zones d'arrière-plan de l'image, recevant ainsi une image constituée de zones dans lesquelles du matériel tissulaire est montré ;
• la division de l'image en régions tumorales et régions saines ;
• l'élimination des régions saines des images ;
• l'introduction de l'image ou de tuiles de l'image dans un modèle de classification ;
• la réception en sortie du modèle de classification, pour l'image ou chaque tuile de l'image, d'une information indiquant à laquelle d'un nombre de classes l'image ou la tuile de l'image appartient, le nombre de classes comprenant au moins deux classes, une première classe et une deuxième classe, la première classe indiquant la présence d'un cancer à fusion NTRK dans l'image ou la tuile de l'image, la deuxième classe indiquant la non-présence d'un cancer à fusion NTRK dans l'image ou la tuile de l'image.

5. Procédé selon la revendication 4, comprenant en outre les étapes suivantes :
• la coloration de zones dans l'image ou dans les tuiles de l'image dans une couleur prédéfinie pour lesquelles la présence d'un cancer à fusion NTRK a été déterminée ;
• la sortie de l'image colorée.

6. Système, comprenant :
un processeur ; et
une mémoire stockant un programme d'application configuré pour effectuer, lorsqu'il est exécuté par le processeur, une opération d'identification d'un ou de plusieurs signes candidats indiquant une fusion oncogène NTRK dans des données patient associées à un patient sujet, l'opération comprenant :
• la réception de données patient d'un patient sujet souffrant d'un cancer, les données patient comprenant au moins une image histopathologique de tissu tumoral du patient sujet ;
• l'introduction des données patient dans un modèle de prédiction entraîné par apprentissage automatique avec un ensemble de données d'entraînement, les données d'entraînement comprenant des images histopathologiques, le modèle de prédiction étant configuré pour identifier dans les données patient une ou plusieurs caractéristiques d'une fusion oncogène NTRK ;
• la réception en sortie du modèle de prédiction d'une valeur de probabilité, la valeur de probabilité indiquant la probabilité que le patient sujet souffre d'un cancer causé par une fusion oncogène NTRK.

7. Support de stockage non transitoire lisible par ordinateur comprenant des instructions exécutables par un processeur permettant d'effectuer une opération d'identification d'un ou de plusieurs signes candidats indiquant une fusion oncogène NTRK dans des données patient associées à un patient sujet, l'opération comprenant :
• la réception de données patient d'un patient sujet souffrant d'un cancer, les données patient comprenant au moins une image histopathologique de tissu tumoral du patient sujet ;
• l'introduction des données patient dans un modèle de prédiction entraîné par apprentissage automatique avec un ensemble de données d'entraînement, les données d'entraînement comprenant des images histopathologiques, le modèle de prédiction étant configuré pour identifier dans les données patient une ou plusieurs caractéristiques d'une fusion oncogène NTRK ;
• la réception en sortie du modèle de prédiction d'une valeur de probabilité, la valeur de probabilité indiquant la probabilité que le patient sujet souffre d'un cancer causé par une fusion oncogène NTRK.
